# EUROPEAN PATENT APPLICATION

(11) **EP 1 938 750 A2**
(43) Date of publication of application: **02.07.2008**
(21) Application number: 07024258.1
(22) Date of filing: 14.12.2007
(51) Int. Cl.: A61B 5/145, A61B 10/00

(54) **Method of therapeutic drug monitoring**

(30) Priority: 21.12.2006 US 876357 P
(71) Applicant: Bayer HealthCare, LLC, Tarrytown, NY 10591 (US)
(72) Inventor: Rebec, Mihailo V., Bristol, Indiana 46507 (US)
(74) Representative: Linhart, Angela

(57) **Abstract**

A method of using a diffusion-based, continuous-monitoring system to monitor the effectiveness of delivering a drug includes creating and maintaining a diffusion channel in an area of skin. The levels of the drug, metabolite, or affected substance of the drug are continuously monitored in the area of the skin for a desired duration via a diffusion-based, continuous-monitoring device. The levels of the drug, the metabolite, or affected substance are analyzed to determine the effectiveness of delivering the therapeutic drug.

## Description

### FIELD OF THE INVENTION

The present invention relates generally to a method of therapeutic drug monitoring and, more specifically, to a method of diffusion-based, continuous therapeutic drug monitoring.

### BACKGROUND OF THE INVENTION

For many years, therapeutic drugs have been used to assist individuals in their healing. The effect of delivering the therapeutic drugs, however, often varies between individuals. For example, the effect of delivering the therapeutic drugs may vary in aspects such as how long does the drug acts for on an individual and how does the drug react with that individual. Because of this variation, some individuals are individually monitored. This monitoring process is referred to as therapeutic drug monitoring (tdm). Therapeutic drug monitoring, if performed, typically occurs with new medication to an individual. One existing method of therapeutic drug monitoring is by repeated taking and testing of a blood sample for the drug of interest. This experience can be unpleasant and very painful for individuals, especially if there is extensive sampling of the blood.

It is desirable to have a method of therapeutic drug monitoring (tdm) that reduces the unpleasantness to those individuals who are being tested, while still providing information on the effect of delivering the therapeutic drug to the individual.

### SUMMARY OF THE INVENTION

According to one method, a diffusion-based, continuous-monitoring system is used to monitor the effectiveness of delivering a therapeutic drug. The method includes creating at least one diffusion channel in an area of skin. The at least one diffusion channel is maintained for a desired duration. The levels of the therapeutic drug, the levels of a metabolite of the therapeutic drug, or the levels of a substance that is affected by the therapeutic drug in the area of the skin are continuously monitored for the desired duration via a diffusion-based, continuous-monitoring device. The levels of the therapeutic drug, the metabolite of the therapeutic drug or the substance that is affected by the therapeutic drug is analyzed so as to determine the effectiveness of delivering the therapeutic drug.

According to another method, a diffusion-based, continuous-monitoring system is used to monitor the effectiveness of delivering a therapeutic drug. The method includes creating at least one diffusion channel in an area of skin. A hydrogel or liquid is topographically applied on the skin to assist in enhancing the diffusion of the therapeutic drug, a metabolite of the therapeutic drug, or a substance that is affected by the therapeutic drug. The at least one diffusion channel is maintained for a desired duration. A diffusion-based, continuous monitoring device is positioned in communication with the hydrogel or liquid. The levels in the skin of the therapeutic drug, the levels of a metabolite of the therapeutic drug, or the levels of a substance that is affected by the therapeutic drug is continuously monitored in the area of the skin via the diffusion-based, continuous monitoring device. The levels of the therapeutic drug, the metabolite of the therapeutic drug or the substance that is affected by the therapeutic drug is analyzed so as to determine the effectiveness of delivering the therapeutic drug.

According to a further method, a diffusion-based, continuous-monitoring system is used to monitor the effectiveness of delivering a therapeutic drug. A diffusion-based, continuous-monitoring device is provided and includes a communications interface that is adapted to connect with a receiving module via a communications link. At least one diffusion channel is created in an area of skin. The at least one diffusion channel is maintained for a desired duration. The levels of the therapeutic drug, the levels of a metabolite of the therapeutic drug, or the levels of a substance that is affected by the therapeutic drug is continuously monitored in the area of the skin for the desired duration via the diffusion-based, continuous-monitoring device. The levels of the therapeutic drug, the metabolite of the therapeutic drug, or the substance that is affected by the therapeutic drug is analyzed so as to determine the effectiveness of delivering the therapeutic drug.

### BRIEF DESCRIPTION OF THE DRAWINGS

FIG. 1 is a diffusion-based, continuous-monitoring system shown in a transdermal application according to one embodiment.

FIG. 2 is the continuous-monitoring system of FIG. 1 being connected to a receiving module.

### DETAILED DESCRIPTION OF THE ILLUSTRATED EMBODIMENTS

The present invention is directed to a method of using a diffusion-based, continuous-monitoring system to monitor the effectiveness of delivering a therapeutic drug. By monitoring and characterizing the drug, doses of the therapeutic drug can be tailed to the individual. Thus, instead of providing the therapeutic drug in a greater dose than necessary (i.e., an overdose), an effective dose is provided to the individual after monitoring and evaluating the effectiveness of delivering the therapeutic drug. Thus, it is desirable to optimize the therapeutic drug process using information from the therapeutic drug monitoring. By optimizing the therapeutic drug being taken, the drug can be safer to the individual by using an effective amount of the same. Additionally, there are typically economic savings to most individuals because the amount of drug being taken is typically reduced. The drugs are tested in, for example, body fluids like ISF (interstitial fluid), whole blood sample, intracellular and intercellular fluids.

The therapeutic drugs to be monitored in the present invention are not limited to a specific delivery mechanism. For example, in one method, the therapeutic drug is administered via an IV. In another method, the therapeutic drug is administered by an IM injection. In a further method, the therapeutic drug is orally administered. In yet another method, the therapeutic drug is administered via a transdermal patch system or via iontophoresis. It is contemplated that the therapeutic drugs may be administered by other techniques.

The therapeutic drugs to be monitored are typically water-soluble drugs. Non-limiting examples of water-soluble drugs include aspirin, Tylenol®, selected antibiotics (e.g., ampicillin and nalidixic acid), and selected chemotherapy drugs (e.g., transplatin complexes). It is contemplated that other types of water-soluble drugs may be monitored using the inventive methods.

Additionally, it is contemplated that other types of therapeutic drugs besides drugs with a high solubility in water may be used in the present invention. Non-limiting examples of water-insoluble drugs include diogoxin, most antibiotics and some chemotherapy drugs. These water-insoluble drugs typically have a very limited water solubility. It is contemplated that other types of water-insoluble drugs may be monitored using the inventive methods.

In one method, the delivery level of the therapeutic drug itself may be continuously monitored. In another method, a metabolite of the delivered therapeutic drug may be continuously monitored. A metabolite includes any product that is metabolized from the therapeutic drug. The metabolite product may be due to the addition to the drug or a breakdown of the drug chemical structure. Thus, the metabolite is in a different form than the therapeutic drug itself. In a further method, an effect of the therapeutic drug to be delivered may be continuously monitored. Thus, the effectiveness of delivering a therapeutic drug may include continuously monitoring the level of the therapeutic drug itself, a metabolite of the therapeutic drug, and/or a substance affected by the therapeutic drug.

The term "level" is defined herein as including any information related to the amount, relative concentration, absolute concentration and ratios of the therapeutic drug, metabolite and the substance affected by the therapeutic drug to assist in determining the efficacy of delivering the drug. The term "level" as defined herein also includes changes in the amount, relative and absolute concentrations, and ratios whether in a percentage or absolute context. These "level" changes may be used over a selected duration of time such as, for example, a time change in amount, concentration or ratio. The "level" may refer to a time change in amount, concentration or ratio and compared to a later time change.

In one example, a cholesterol-reducing drug may be tested for "levels" by measuring the absolute cholesterol values, ratios of good and bad cholesterol, percentage change of the cholesterol values and concentrations of the cholesterol values. In another example, the level may refer to the change over a duration (e.g., 5 minutes) between the drug and its metabolites and a later duration (e.g., 5 minutes) at later time.

Examples of metabolite products include, but are not limited to, many longer-acting drugs. Typically, longer-acting drugs are introduced into the body in a blocked-on active form until the body reacts with the drug to reach an active form. Thus, the active form, which is a drug metabolite, is monitored to provide the effectiveness of the delivery of the blocked-on active form of the drug. Another examples is encapsulating a drug to obtain a slow release that can be monitored. Another example of a metabolite product is a longer-acting insulin.

A further type of example is a drug that has therapeutic action and also has metabolites with therapeutic properties. One example of such a drug is valproic acid and its metabolite 2-N-propyl-3-ketopentanoic acid. Another example is mephobarbital, which has some therapeutic action, and its metabolite phenobarbital, which over time is produced by the liver. Thus, in these embodiments, the monitoring of the metabolite can be as important as monitoring of the drug itself.

As discussed above, the effect of a therapeutic drug may be continuously monitored. For example, the effectiveness of delivering a therapeutic drug (e.g., insulin) may be determined by continuously monitoring another substance (e.g., glucose). If the insulin is being properly delivered, then the levels of glucose should decrease. In another example, the effectiveness of delivering a cholesterol-reducing drug may be determined by continuously monitoring the cholesterol level in the skin. In another example, the effectiveness of delivering an anticoagulant drug may be determined by continuously monitoring the coagulation itself. In a further example, the effectiveness of delivering antihistamines may be determined by continuously monitoring the histamines.

According to one method, at least three criteria may be considered in selecting a suitable diffusion-based, continuous-monitoring system to evaluate the effectiveness of delivering a therapeutic drug in a body fluid sample from an area of skin. First, a diffusion-enhancing process for the skin is selected. Second, a material is selected to assist in maintaining contact with the skin and further enhance diffusion of the therapeutic drug from the skin. Third, a diffusion-based, continuous-monitoring system is selected to determine the effectiveness of delivering the therapeutic drug of the body fluid sample that is diffused from the skin.

According to one method, the diffusion-enhancing process for the skin is selected based on factors such as the following: length of time of testing, the therapeutic drug/ metabolite /affected substance to be monitored, and the area of the skin from where the therapeutic drug/metabolite /affected substance is located. It is desirable for the diffusion-enhancing process to maintain the diffusion channel throughout the desired time period.

Skin abrasion is typically selected when the continuous-testing period is a relatively short period of time (e.g., less than about 8 hours). Skin abrasion is desirable for a shorter continuous-testing period because of the minimum impact on the skin. It is contemplated that a number of skin-abrasion techniques may be used. In one technique, skin abrasion occurs using a gel material including pumas or other skin-abrasion materials. In this technique, the gel material including pumas or other skin-abrasion materials is rubbed on the skin to increase the permeability of the skin. Skin abrasion may occur by other techniques such as using a generally coarse material (e.g., sandpaper), tape peeling or pumas paper.

To increase the porosity of skin (e.g., the stratum comium, epidermis and/or dermis), chemical agents and physical agents may be used. The chemical and physical agents desirably assist in breaking down the lipids on the stratum cornium. The chemical and physical agents are typically used in short-term solutions and medium-term solutions. It is contemplated, however, that the chemical and physical agents may be used in long-term solutions.

The chemical agents may be skin hydration or skin exfoliates that increase the hydration and porosity of the skin. Skin hydration/exfoliates may include those commercially used in skin products. Some non-limiting examples of chemical agents that may be used include d-limonene, L-limonene, and alpha-terpinene. These chemical agents act by extracting lipids from, for example, the stratum comium, which disrupts the stratum cornium and desquamates stratum cornium flake.

There are number of physical processes that can be used to enhance the permeability of the skin so as to increase the diffusion of the monitored drug/metabolite/affected substance of interest. In one process, needle-less jet injectors are used with very fine, particulates of inert material that are fired directly into the skin using high-pressure gas. In another process, pulsed magnetic fields may be used to create transient pores in the skin, resulting in increased permeation. It is contemplated that other physical processes may be used to enhance the permeability of the skin.

If the continuous-testing period is longer (e.g., from about 8 hours to 24 hours), then a different diffusion-enhancing approach may be selected. For such a period, various approaches may be selected such as microporation, microneedle-diffusion enhancement, pressure members, multiple lances, heavier abrasions and ultrasound energy.

In one method, a microporation or a microneedle-diffusion enhancement approach may be used for longer continuous testing periods. A microporation approach creates sub-millimeter size apertures in the epidermis. In one microporation technique, a laser-poration technique may be used to deliver laser power directly to the skin to create apertures or pores. Laser-poration techniques are typically used to form shallow apertures or pores.

In a further method, a series of absorbing dots is located in the stratum cornium and then followed by delivery of a laser that absorbs and softens at each point. The absorbent material converts the laser power to heat, which combined with pressure, create the apertures in the stratum cornium.

A microneedle-diffusion enhancement approach creates apertures in the epidermis and dermis. In another method, a pressure member is adapted to apply pressure to and stretch the skin in preparation for forming a tear in the skin. In another approach, a heavier abrasion of the skin could be performed such as using a more coarse material. An example of a more coarse material includes, but is not limited to, coarser sandpaper.

In another method, ultrasound energy is used to disrupt the lipid bilayer of the stratum cornium so as to increase the skin permeability. Ultrasound energy typically forms shallow apertures. By increasing the skin permeability, the amount of interstitial fluid (ISF) used in monitoring the delivering of the therapeutic drug/drug metabolite is increased. One non-limiting source of an ultrasound energy system is Sontra SonoPrep® ultrasonic skin permeation system marketed by Sontra Medical Corporation. The SonoPrep® system applies relatively low frequency ultrasonic energy to the skin for a limited duration (from about 10 to 20 seconds). The ultrasonic horn contained in the device vibrates at about 55,000 times per second (55KHz) and applies energy to the skin through the liquid medium (e.g., hydrogel or liquid) to create cavitation bubbles that expand and contract in the liquid medium.

The chemical and physical agents discussed above in the generally short term may also be used in medium continuous-testing periods to increase and maintain the porosity of the skin. It is contemplated, however, that the chemical and physical agents may be used to obtain longer term action. For example, delipidating agents may be used in combination with physical agents such as ultrasonic preparation to create more long term diffusional channels.

If the continuous-testing period is even longer (e.g., at least 24 hours to about 48 hours), a deep, laser-ablation technique or lance may be selected. A deep, laser-ablation technique is desirable because the monitoring process can function longer due to the time needed to close the aperture created in the skin. The laser-ablation technique typically forms wide apertures. It is contemplated that a microneedle diffusion-enhancing approach, laser poration or lancets may also be used to provide a deeper aperture.

The size of the therapeutic drug/metabolite/affected substance to be monitored may also affect the diffusion-enhancing technique to be used. For example, if the therapeutic drug/metabolite/affected substance to be monitored is a larger molecule (e.g., vaccines and antibodies), then the diffusion-enhancing process would desirably form a larger aperture in the skin. Similarly, if the delivery of a smaller molecule is to be monitored, the diffusion-enhancing process desirably would form a smaller aperture in the skin. Most of the therapeutic drugs/metabolites/affected substances to be monitored have smaller molecular weights so therefore it is not necessary to form larger apertures.

The area of the skin where the desired therapeutic drug/metabolite/affected substance is located is also a consideration in selecting the diffusion-enhancing process. For example, if the epidermis or the upper part of the dermis is where the therapeutic drug/metabolite/affected substance is to be monitored, the diffusion-enhancing process would be selected to disrupt the stratum cornium. Examples of such diffusion-enhancing processes include skin abrasion, skin hydrations (which increase the hydration of the skin), and skin exfoliates.

If monitoring of the therapeutic drug/metabolite/affected substance in the ISF of the lower dermis is desired, the diffusion-enhancing process is selected to create at least one diffusion channel deep into the dermis. If monitoring of the therapeutic drug/metabolite/affected substance in the ISF in the subcutaneous region is desired, the diffusion-enhancing process is selected to create at least one diffusion channel through the dermis into the subcutaneous region. Non-limiting examples of diffusion-enhancing processes that create at least one deep diffusion channel into the dermis or subcutaneous region include, but are not limited to, laser poration, microneedles and lancets. It is also contemplated that an electric discharge with high energy and conductivity may also be used to create at least one deep diffusion channel.

The chemical and physical agents discussed above in the generally short term may also be used in longer continuous-testing periods to increase and maintain the porosity of the skin.

In addition to selecting a continuous diffusion-enhancing method, a material is selected to assist in maintaining contact with the skin and to match the monitoring requirements in one method. The diffusion-enhancing material maintains desirable skin contact at all times and assists in maintaining the diffusion channel. The material may be selected based on factors such as the following: length of monitoring time, the therapeutic drug/metabolite/affected substance to be monitored, and the area of the skin from which the drug/metabolite/affected substance is located. For example, the viscosity of the material may be matched with the therapeutic drug/metabolite/affected substance to be monitored.

Examples of diffusion-enhancing materials that may be used in the diffusion-based, continuous-monitoring system include, but are not limited to, hydrogels, liquids and a liquid-stabilizing layer containing a liquid or hydrogel. The diffusion-enhancing material also desirably assists in hydrating the skin and maintaining an opening in the skin. By maintaining the opening, a liquid bridge is formed such that the therapeutic drug/metabolite/affected substance diffuses from a layer in the skin through the opening. The liquid bridge may be between a hydrogel/liquid and a body fluid such as ISF (interstitial fluid) or a whole blood sample.

Hydrogels typically have high water content and tacky characteristics. Hydrogels assist in carrying the therapeutic drug/metabolite/affected substance to the continuous-monitoring system and also assist in hydrating the skin. Hydrogels are typically used with smaller sized drug/metabolite/affected substance molecules, shorter analysis times and an upper dermis analysis site.

A hydrogel composition is defined herein as including a cross-linked polymer gel. The hydrogel composition generally comprises at least one monomer and a solvent. The solvent is typically substantially biocompatible with the skin. Non-limiting examples of solvents that may be used in the hydrogel composition include water and a water mixture. The amount of solvent in the hydrogel is generally from about 10 to about 95 weight percent and may vary depending on the monomer amount, cross linking, and/or the desired composition of the gel. One non-limiting example of a hydrogel/liquid is dimethylsulfoxide (DMSO). DMSO also assists in solubilizing lipids. An example of a liquid that may be used includes an alcohol (e.g., glycerol) in combination with water. The chemical agents discussed above may be added to the hydrogel composition to maintain the porosity of the skin. It is contemplated that other hydrogels/liquids may be used.

The hydrogel/liquid may be located in a material (i.e., a liquid-stabilizing layer). This material may be selected to assist in maintaining contact with the skin as well as being able to retain the hydrogel/liquid. The liquid-stabilizing layer may include a chamber where the therapeutic drug/metabolite/affected substance of interest can diffuse. One non-limiting example of a material that can be used is a sponge or spongy material. The spongy material includes unbound liquid such as water and provides some structure to the unbound water. The spongy material is typically used with larger sized therapeutic drug/metabolite/affected substance molecules, longer monitoring times and deeper monitoring sites.

Other materials may be used to create content with skin and conduct further analysis. Materials include, but are not limited to, woven materials, non-woven materials, and polymeric films with apertures or porations formed therein. The polymeric films may be, for example, cast polymeric films. These materials may be used with liquids to facilitate diffusion of the material from the skin.

The amount of hydrogel that is selected is based on the need to provide a hydrated skin and having the hydrogel remain in intimate contact with the skin. One disadvantage of using a large amount of hydrogel is the potential impact on the lag time of the therapeutic drug/metabolite/affected substance diffusing to the diffusion-based, continuous-monitoring system and, thus, the potential impact on the analysis time.

Additives may be added to the hydrogel or liquid. For example, to assist in dissolving lipids, the hydrogel or liquid may include SDS (sodium dodecyl (lauryl) sulfate) or SLS (sodium lauryl (laureth) sulfate). It is contemplated that other additives may be included in the hydrogel or liquid to assist in dissolving the lipids such as soaps. In another embodiment, DMSO may be used as an additive to another hydrogel/liquid to assist in solubilizing lipids.

Additional analysis components may also be added to the hydrogels/liquids. More specifically, additives may be added to the hydrogels/liquid to assist in monitoring the delivery of the therapeutic drug/metabolite/affected substance. In one embodiment, an enzyme is added to the hydrogel or liquid.

In another embodiment, an interference-filtering component may be added to the hydrogels/liquids. These interference-filtering components may include size exclusion, interference-binding molecules, and/or molecules that remove or convert interfering substances. Some non-limiting examples of interference-binding molecules are antibodies or materials with appropriate charges. Another example is changing the ionic charge nature of the hydrogel or diffusion matrix such that charged interference molecules are inhibited from getting to the surface of the continuous-monitoring device.

Hypertonic solutions, hypotonic solutions and buffered solutions may be used as a diffusion-enhancing material. Hypertonic solutions are solutions having a high solute concentration, while hypotonic solutions are solutions having a low solute concentration. Hypertonic solutions assist in driving up the body fluid (e.g., ISF) closer to the skin surface. Hypotonic solutions, on the other hand, assist in driving up the therapeutic drug/metabolite/affected substance closer to the skin surface. The hypertonic or hypotonic solutions in one embodiment may be included with the hydrogel or liquid.

To assist in monitoring the delivery level of the therapeutic drug/metabolite/affected substance, a charged additive may be added to the hydrogel or liquid. In one embodiment, a cationic surfactant is added to the hydrogel or liquid. In another example, an anionic surfactant is added to the hydrogel or liquid. One approach is to add an organic molecule (e.g., methanol) as a component of the hydrogel/liquid. The addition of the organic molecule increases the likelihood that the therapeutic drug/metabolite/affected substance are more hydrophobic would be extracted/diffused in the hydrogel/liquid. In another approach, a screen or another conducting surface is placed on the gel to create a charge that attracts positively or negatively charged therapeutic drugs/metabolites/affected substances.

It is contemplated that other additives such as anticoagulants and/or buffers may be used to assist in maintaining the localized pH near the optimal level.

It is contemplated that other additives may be added to the hydrogel or liquid to assist in monitoring the effectiveness of the delivery of the therapeutic drug/metabolite/affected substance.

A diffusion-based, continuous-monitoring device is selected that monitors the therapeutic drug level, the drug metabolite level, or the affected substance level of the body fluid sample that is diffused from the skin. The diffusion-based, continuous-monitoring device may be selected from an electrochemical-monitoring system, an optical-monitoring system, an osmotic-monitoring system, or a pressure-based monitoring system. A pressure-based monitoring system includes systems associated with the binding of an analyte by components of the hydrogel, which results in a volume change in the gel. The monitoring may be performed in a vertical or horizontal direction with respect to the diffusion channel(s) formed in the skin. It is contemplated that the therapeutic drug/metabolite/affected substance may be carried out in the material that is selected to assist in maintaining contact with the skin (e.g., the hydrogel or liquid).

The diffusion-based, continuous-monitoring device is typically located near or at the skin. The diffusion-based, continuous-monitoring device may be coupled with the skin and is typically in intimate contact with the skin. For example, the diffusion-based, continuous-monitoring device may be adhered to the skin with an adhesive. The adhesive may be the hydrogel itself. In another embodiment, the adhesive is a separate component whose sole function is to adhere the continuous-monitoring device to the skin. In a further method, the diffusion-based, continuous-monitoring device may be coupled to the skin by a mechanical attachment. For example, the mechanical attachment may be a wrist band (e.g., an elastic band, a watch band, a band with an attachment mechanism such as a hook and loop mechanism). One example of a hook and loop mechanism is a Velcro® strap marketed by 3M Corporation of St. Paul, Minnesota. It is contemplated that other mechanical attachments may be used to couple or attach the continuous-monitoring device with skin.

The diffusion-based, continuous-monitoring device may have a variety of forms. For example, the continuous-monitoring device may be a pad, circular disk, polygonal shaped or non-polygonal shaped. The continuous-monitoring system may include an analysis element. For example, a pad with the analysis element may be used instead of, or in addition to, the analysis element being initially located in the hydrogel or liquid. In one embodiment, an enzyme may be initially located in the continuous-monitoring device.

In one embodiment, the diffusion-based, continuous-monitoring device includes a processor to process the data, a memory that stores data, and a communications interface. The data may be stored at regular intervals such as, for example, every minute, every 5 minutes or every 30 minutes. The intervals may be shorter such as every second or longer such as being several hours apart. The desired intervals depend on the rate of change on the therapeutic drug/metabolite/affected substance. Some drugs (such as those administered via an IV) have very short half lives and may desirably collect data every second. Other drugs (such as those administered intramuscular) are absorbed slowly and do not metabolize quickly may desirably collect data in hourly intervals. It is contemplated that other regular or non-regular intervals may be used to store the data.

The data may be any information that assists in monitoring the effectiveness of delivering the therapeutic drug. This information may include the level of the therapeutic drug, the level of a therapeutic metabolite, or the effect of the therapeutic drug (e.g., the level of another compound affected by the therapeutic drug). Other data may include effectiveness of the drug that is being used such that pharmacodynamic data is tabulated. This information may then be processed to determine a recommended level of drug with a desired efficacy. By storing the data in the continuous-monitoring device, this data can be accessed and used to assist in monitoring the effectiveness of delivering the therapeutic drug. It is desirable for the continuous-monitoring device to tabulate, transmit and store information that assists in determining the effectiveness of delivering the therapeutic drug.

In one embodiment, the continuous-monitoring device is connected to a remote-monitoring system over a communications link. The communications link between the continuous-monitoring device and the remote-monitoring system may be wireless, hard wired or a combination thereof. The wireless communications link may include an RF link, an infrared link or an inductive magnetic link. The wireless implementation may include an internet connection. The continuous-monitoring device may communicate via its communication interface with devices such as a computer, e-mail server, cell phone or telephone. It is contemplated that the continuous-monitoring device may include other devices that are capable of storing, sending and/or receiving information.

The remote-monitoring system enables an individual such as a physician to monitor the effectiveness of delivering the therapeutic drug from a remote location. The remote-monitoring system may be located in, for example, a hospital. The physician may be able to access information from the continuous-monitoring device via its communications interface using, for example, a computer or telephone. The remote-monitoring system is especially desirable for patients who are less lucid and need assistance with monitoring the effectiveness of delivering the therapeutic drugs. A remote-monitoring system may also assist in compliance of an individual taking a therapeutic drug. It is desirable for the remote-monitoring system to be able to display, calibrate and store information received from the continuous-monitoring device.

The remote-monitoring system may be used to send back instructional information to the patients. The remote-monitoring system may be used to automatically adjust the doses of the therapeutic drug of the patient based on the feedback of the monitoring of the therapeutic drug/metabolite/affected substance. In such an embodiment, diffusion-based continuous-monitoring device includes a communications link that has a receiver component to receive instructions from the remote-monitoring system in addition to a transmitter component to transmit information to the remote-monitoring system.

In one method, the continuous-monitoring device may forward information over a communications link in real-time. In another method, the continuous-monitoring device may store and process the data before forwarding the information over a communications link in another embodiment.

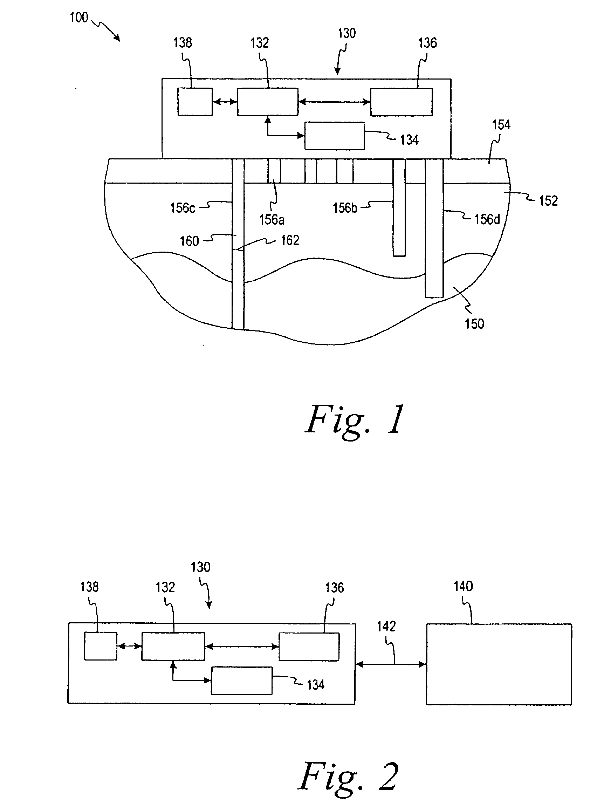

Referring to FIG. 1, a diffusion-based, continuous-monitoring system 100 is shown in a transdermal application. The continuous-monitoring system 100 includes a continuous-monitoring device 130 being placed above skin. The continuous-monitoring device 130 of FIG. 1 includes a processor 132, memory 134, a communication interface 136 and an analysis component 138. Referring to FIG. 2, the continuous-monitoring device 130 is shown in communication with a receiving module 140 (e.g., a remote-monitoring station) over a communications link 142.

The skin as shown in FIG. 1 includes a dermis layer 150, an epidermis layer 152 and a stratum cornium layer 154. The stratum cornium layer 154 has a plurality of channels 156a-d formed therein. The plurality of channels 156a-d may be formed by different methods such as discussed above. The channels may be of different sizes and depths depending on the drug/drug metabolite/affected substance to be monitored and the location of the drug/drug metabolite/affected substance in the skin. The therapeutic drug/drug metabolite/affected substance of interest may be located in the different layers of the skin. For example, the therapeutic drug/drug metabolite/affected substance may be located in the dermis layer 150, the epidermis layer 152, stratum cornium layer 154 or subcutaneous fat layer (not shown in FIG. 1).

In one method, a hydrogel/liquid is used to assist in diffusing the therapeutic drug/drug metabolite/affected substance to the surface of the skin. The channel 156c is shown with hydrogel/liquid 160. An interface 162 is formed between the hydrogel/liquid and the body fluid. The analysis may be performed in several locations in the continuous-monitoring system 100. For example, the analysis may be performed using the analysis components 138 in the continuous-monitoring device 130. The analysis components may include components such as a sensor, an enzyme or reagent, potentiostat, electrochemical analysis components (e.g., plurality of electrodes, etc.) and/or optical analysis components (e.g., light source, detector, etc.). In another example, the analysis may be performed on the skin and/or in the channels. It is contemplated that the analysis may take place in more than one location. For example, the hydrogel/liquid may include an analysis portion (e.g., a reagent or enzyme) that reacts with therapeutic drug/drug metabolite/affected substance in the channel, while the remainder of the analysis takes place on the skin or in the continuous-monitoring device 130.

According to one process, a technician programs the diffusion-based, continuous-monitoring device for operation. The technician may program, for example, the therapeutic drug, drug metabolite or affected substance to be monitored, the length of time of the monitoring, the type of drug, drug metabolites/affected substance and when the device can be removed. For example, insulin or glucose may be monitored to determine the effectiveness of an oral type II diabetes drug. The technician may then proceed to form apertures in the skin that function as diffusion channels as discussed above for the desired time period. The technician locates the continuous-monitoring device on the individual. In one method, the technician locates the continuous-monitoring device on the arm. It is contemplated that the technician may locate the continuous-monitoring device on other locations. The continuous-monitoring device is adapted to process, calibrate, display, store and/or transmit information related to the therapeutic drug, drug metabolite, or affected substance. It is also contemplated that continuous-monitoring device may receive information or direction pertaining to a drug-delivery system such as an IV pump.

### PROCESS A

A method of using a diffusion-based, continuous-monitoring system to monitor the effectiveness of delivering a therapeutic drug, the method comprising the acts of:
creating at least one diffusion channel in an area of skin;
maintaining the at least one diffusion channel for a desired duration;
continuously monitoring the levels of the therapeutic drug, the levels of a metabolite of the therapeutic drug or the levels of a substance that is affected by the therapeutic drug in the area of the skin for the desired duration via a diffusion-based, continuous-monitoring device; and
analyzing the levels of the therapeutic drug, the metabolite of the therapeutic drug or the substance that is affected by the therapeutic drug so as to determine the effectiveness of delivering the therapeutic drug.

### PROCESS B

The method of process A wherein the at least one diffusion channel is a plurality of diffusion channels.

### PROCESS C

The method of process A wherein the at least one diffusion channel is created by skin abrasion, microporation, microneedle-diffusion enhancement, pressure members, a lancet, ultrasound energy or laser ablation.

### PROCESS D

The method of process C wherein the at least one diffusion channel is created by laser ablation.

### PROCESS E

The method of process A wherein the process is continuously monitored for at least 8 hours.

### PROCESS F

The method of process E wherein the process is continuously monitored for at least 24 hours.

### PROCESS G

The method of process A wherein the diffusion-based, continuous-monitoring system is an electrochemical-monitoring system.

### PROCESS H

The method of process A wherein the diffusion-based, continuous-monitoring system is an optical-monitoring system.

### PROCESS I

The method of process A wherein the levels of the therapeutic drug are continuously monitored.

### PROCESS J

The method of process A wherein the levels of a metabolite of the therapeutic drug are continuously monitored.

### PROCESS K

The method of process A wherein the levels of a substance that is affected by the therapeutic drug are continuously monitored.

### PROCESS L

The method of process K wherein the substance is glucose and the therapeutic drug is insulin.

### PROCESS M

The method of process A further including storing the levels of the therapeutic drug, the metabolite of the therapeutic drug or the substance that is affected by the therapeutic drug.

### PROCESS N

The method of process A further including displaying the levels of the therapeutic drug, the metabolite of the therapeutic drug or the substance that is affected by the therapeutic drug.

### PROCESS O

The method of process A wherein the therapeutic drug is a water-soluble drug.

### PROCESS P

The method of process A wherein the therapeutic drug is a water-insoluble drug.

### PROCESS Q

A method of using a diffusion-based, continuous-monitoring system to monitor the effectiveness of delivering a therapeutic drug, the method comprising the acts of:
creating at least one diffusion channel in an area of skin;
topographically applying a hydrogel or liquid on the skin to assist in enhancing the diffusion of the therapeutic drug, a metabolite of the therapeutic drug or a substance that is effected by the therapeutic drug;
maintaining the at least one diffusion channel for a desired duration;
positioning a diffusion-based, continuous monitoring device in communication with the hydrogel or liquid;
continuously monitoring the levels of the therapeutic drug, the levels of a metabolite of the therapeutic drug or the levels of a substance that is affected by the therapeutic drug in the area of the skin via the diffusion-based, continuous monitoring device; and
analyzing the levels of the therapeutic drug, the metabolite of the therapeutic drug or the substance that is affected by the therapeutic drug so as to determine the effectiveness of delivering the therapeutic drug.

### PROCESS R

The method of process Q wherein the hydrogel or liquid includes a diagnostic element to assist in analyzing the levels of the therapeutic drug, the metabolite of the therapeutic drug or the substance that is affected by the therapeutic drug.

### PROCESS S

The method of process Q wherein positioning the monitoring device includes attaching the monitoring device to the skin.

### PROCESS T

The method of process Q further including transmitting the levels of the therapeutic drug, the metabolite of the therapeutic drug or the substance that is affected by the therapeutic drug to a receiving module.

### PROCESS U

The method of process Q wherein the at least one diffusion channel is created by skin abrasion, microporation, microneedle-diffusion enhancement, pressure members, a lancet, ultrasound energy or laser ablation.

### PROCESS V

The method of process U wherein the at least one diffusion channel is created by a laser ablation.

### PROCESS W

The method of process Q wherein the process is continuously monitored for at least 8 hours.

### PROCESS X

The method of process W wherein the process is continuously monitored for at least 24 hours.

### PROCESS Y

The method of process Q wherein the diffusion-based, continuous-monitoring system is an electrochemical-monitoring system.

### PROCESS Z

The method of process Q wherein the diffusion-based, continuous-monitoring system is an optical-monitoring system.

### PROCESS AA

The method of process Q wherein the levels of the therapeutic drug are continuously monitored.

### PROCESS BB

The method of process Q wherein the levels of a metabolite of the therapeutic drug are continuously monitored.

### PROCESS CC

The method of process Q wherein the levels of a substance that is affected by the therapeutic drug are continuously monitored.

### PROCESS DD

The method of process CC wherein the substance is glucose and the therapeutic drug is insulin.

### PROCESS EE

The method of process Q further including storing the levels of the therapeutic drug, the metabolite of the therapeutic drug or the substance that is affected by the therapeutic drug.

### PROCESS FF

The method of process Q further including displaying the levels of the therapeutic drug, the metabolite of the therapeutic drug or the substance that is affected by the therapeutic drug.

### PROCESS GG

The method of process Q wherein the therapeutic drug is a water-soluble drug.

### PROCESS HH

The method of process Q wherein the therapeutic drug is a water-insoluble drug.

### PROCESS II

A method of using a diffusion-based, continuous-monitoring system to monitor the effectiveness of delivering a therapeutic drug, the method comprising the acts of:
providing a diffusion-based, continuous-monitoring device, the device including a communications interface that is adapted to connect with a receiving module via a communications link;
creating at least one diffusion channel in an area of skin;
maintaining the at least one diffusion channel for a desired duration;
continuously monitoring the levels of the therapeutic drug, the levels of a metabolite of the therapeutic drug or the levels of a substance that is affected by the therapeutic drug in the area of the skin for the desired duration via the diffusion-based, continuous-monitoring device; and
analyzing the levels of the therapeutic drug, the metabolite of the therapeutic drug or the substance that is affected by the therapeutic drug so as to determine the effectiveness of delivering the therapeutic drug.

### PROCESS JJ

The method of process II further including transmitting information directed to the levels of the therapeutic drug, the levels of a metabolite of the therapeutic drug or the levels of a substance that is affected by the therapeutic drug to the receiving module via the communications link.

### PROCESS KK

The method of process JJ further including receiving instructions from the receiving module via the communications link directed to the deliver of the therapeutic drug.

### PROCESS LL

The method of process JJ wherein the transmitting of information is performed on a wireless system.

### PROCESS MM

The method of process JJ wherein the transmitting of information is performed on a wired system.

### PROCESS NN

The method of process JJ wherein the transmitted information occurs at intervals between 5 minutes and 2 hours.

### PROCESS OO

The method of process II wherein the at least one diffusion channel is created by skin abrasion, microporation, microneedle-diffusion enhancement, pressure members, a lancet, ultrasound energy or laser ablation.

### PROCESS PP

The method of process OO wherein the at least one diffusion channel is created by the laser ablation.

### PROCESS QQ

The method of process II wherein the process is continuously monitored for at least 8 hours.

### PROCESS RR

The method of process QQ wherein the process is continuously monitored for at least 24 hours

### PROCESS SS

The method of process II wherein the diffusion-based, continuous-monitoring system is an electrochemical-monitoring system.

### PROCESS TT

The method of process II wherein the diffusion-based, continuous-monitoring system is an optical-monitoring system.

### PROCESS UU

The method of process II wherein the levels of the therapeutic drug are continuously monitored.

### PROCESS VV

The method of process II wherein the levels of a metabolite of the therapeutic drug are continuously monitored.

### PROCESS WW

The method of process II wherein the levels of a substance that is affected by the therapeutic drug are continuously monitored.

### PROCESS XX

The method of process WW wherein the substance is glucose and the therapeutic drug is insulin.

### PROCESS YY

The method of process II further including storing the levels of the therapeutic drug, the metabolite of the therapeutic drug or the substance that is affected by the therapeutic drug.

### PROCESS ZZ

The method of process II wherein the therapeutic drug is a water-soluble drug.

### PROCESS AAA

The method of process II wherein the therapeutic drug is a water-insoluble drug.

While the present invention has been described with reference to one or more particular embodiments, those skilled in the art will recognize that many changes may be made thereto without departing from the spirit and scope of the present invention. Each of these embodiments, and obvious variations thereof, is contemplated as falling within the spirit and scope of the invention as defined by the appended claims.

## Claims

1. A method of using a diffusion-based, continuous-monitoring system to monitor the effectiveness of delivering a therapeutic drug, the method comprising the acts of:
creating at least one diffusion channel in an area of skin;
maintaining the at least one diffusion channel for a desired duration;
continuously monitoring the levels of the therapeutic drug, the levels of a metabolite of the therapeutic drug or the levels of a substance that is affected by the therapeutic drug in the area of the skin for the desired duration via a diffusion-based, continuous-monitoring device; and
analyzing the levels of the therapeutic drug, the metabolite of the therapeutic drug or the substance that is affected by the therapeutic drug so as to determine the effectiveness of delivering the therapeutic drug.

2. The method of claim 1, wherein the at least one diffusion channel is a plurality of diffusion channels.

3. The method of claim 1, wherein the at least one diffusion channel is created by skin abrasion, microporation, microneedle-diffusion enhancement, pressure members, a lancet, ultrasound energy or laser ablation.

4. The method of claim 3, wherein the at least one diffusion channel is created by laser ablation.

5. The method of claim 1, wherein the process is continuously monitored for at least 8 hours.

6. The method of claim 5, wherein the process is continuously monitored for at least 24 hours.

7. The method of claim 1, wherein the diffusion-based, continuous-monitoring system is an electrochemical-monitoring system.

8. The method of claim 1, wherein the diffusion-based, continuous-monitoring system is an optical-monitoring system.

9. The method of claim 1, wherein the levels of the therapeutic drug are continuously monitored.

10. The method of claim 1, wherein the levels of a metabolite of the therapeutic drug are continuously monitored.

11. The method of claim 1, wherein the levels of a substance that is affected by the therapeutic drug are continuously monitored.

12. The method of claim 11, wherein the substance is glucose and the therapeutic drug is insulin.

13. The method of claim 1, further including storing the levels of the therapeutic drug, the metabolite of the therapeutic drug or the substance that is affected by the therapeutic drug.

14. The method of claim 1, further including displaying the levels of the therapeutic drug, the metabolite of the therapeutic drug or the substance that is affected by the therapeutic drug.

15. The method of claim 1, wherein the therapeutic drug is a water-soluble drug.

16. The method of claim 1, wherein the therapeutic drug is a water-insoluble drug.

17. A method of using a diffusion-based, continuous-monitoring system to monitor the effectiveness of delivering a therapeutic drug, the method comprising the acts of:
creating at least one diffusion channel in an area of skin;
topographically applying a hydrogel or liquid on the skin to assist in enhancing the diffusion of the therapeutic drug, a metabolite of the therapeutic drug or a substance that is effected by the therapeutic drug;
maintaining the at least one diffusion channel for a desired duration;
positioning a diffusion-based, continuous monitoring device in communication with the hydrogel or liquid;
continuously monitoring the levels of the therapeutic drug, the levels of a metabolite of the therapeutic drug or the levels of a substance that is affected by the therapeutic drug in the area of the skin via the diffusion-based, continuous monitoring device; and
analyzing the levels of the therapeutic drug, the metabolite of the therapeutic drug or the substance that is affected by the therapeutic drug so as to determine the effectiveness of delivering the therapeutic drug.

18. The method of claim 17, wherein the hydrogel or liquid includes a diagnostic element to assist in analyzing the levels of the therapeutic drug, the metabolite of the therapeutic drug or the substance that is affected by the therapeutic drug.

19. The method of claim 17, wherein positioning the monitoring device includes attaching the monitoring device to the skin.

20. The method of claim 17, further including transmitting the levels of the therapeutic drug, the metabolite of the therapeutic drug or the substance that is affected by the therapeutic drug to a receiving module.

21. The method of claim 17, wherein the at least one diffusion channel is created by skin abrasion, microporation, microneedle-diffusion enhancement, pressure members, a lancet, ultrasound energy or laser ablation.

22. The method of claim 21, wherein the at least one diffusion channel is created by a laser ablation.

23. The method of claim 17, wherein the process is continuously monitored for at least 8 hours.

24. The method of claim 23, wherein the process is continuously monitored for at least 24 hours.

25. The method of claim 17, wherein the diffusion-based, continuous-monitoring system is an electrochemical-monitoring system.

26. The method of claim 17, wherein the diffusion-based, continuous-monitoring system is an optical-monitoring system.

27. The method of claim 17, wherein the levels of the therapeutic drug are continuously monitored.

28. The method of claim 17, wherein the levels of a metabolite of the therapeutic drug are continuously monitored.

29. The method of claim 17, wherein the levels of a substance that is affected by the therapeutic drug are continuously monitored.

30. The method of claim 29, wherein the substance is glucose and the therapeutic drug is insulin.

31. The method of claim 17, further including storing the levels of the therapeutic drug, the metabolite of the therapeutic drug or the substance that is affected by the therapeutic drug.

32. The method of claim 17, further including displaying the levels of the therapeutic drug, the metabolite of the therapeutic drug or the substance that is affected by the therapeutic drug.

33. The method of claim 17, wherein the therapeutic drug is a water-soluble drug.

34. The method of claim 17, wherein the therapeutic drug is a water-insoluble drug.

35. A method of using a diffusion-based, continuous-monitoring system to monitor the effectiveness of delivering a therapeutic drug, the method comprising the acts of:
providing a diffusion-based, continuous-monitoring device, the device including a communications interface that is adapted to connect with a receiving module via a communications link;
creating at least one diffusion channel in an area of skin;
maintaining the at least one diffusion channel for a desired duration;
continuously monitoring the levels of the therapeutic drug, the levels of a metabolite of the therapeutic drug or the levels of a substance that is affected by the therapeutic drug in the area of the skin for the desired duration via the diffusion-based, continuous-monitoring device; and
analyzing the levels of the therapeutic drug, the metabolite of the therapeutic drug or the substance that is affected by the therapeutic drug so as to determine the effectiveness of delivering the therapeutic drug.

36. The method of claim 35, further including transmitting information directed to the levels of the therapeutic drug, the levels of a metabolite of the therapeutic drug or the levels of a substance that is affected by the therapeutic drug to the receiving module via the communications link.

37. The method of claim 36, further including receiving instructions from the receiving module via the communications link directed to the deliver of the therapeutic drug.

38. The method of claim 36, wherein the transmitting of information is performed on a wireless system.

39. The method of claim 36, wherein the transmitting of information is performed on a wired system.

40. The method of claim 36, wherein the transmitted information occurs at intervals between 5 minutes and 2 hours.

41. The method of claim 35, wherein the at least one diffusion channel is created by skin abrasion, microporation, microneedle-diffusion enhancement, pressure members, a lancet, ultrasound energy or laser ablation.

42. The method of claim 41, wherein the at least one diffusion channel is created by the laser ablation.

43. The method of claim 35, wherein the process is continuously monitored for at least 8 hours.

44. The method of claim 43, wherein the process is continuously monitored for at least 24 hours

45. The method of claim 35, wherein the diffusion-based, continuous-monitoring system is an electrochemical-monitoring system.

46. The method of claim 35, wherein the diffusion-based, continuous-monitoring system is an optical-monitoring system.

47. The method of claim 35, wherein the levels of the therapeutic drug are continuously monitored.

48. The method of claim 35, wherein the levels of a metabolite of the therapeutic drug are continuously monitored.

49. The method of claim 35, wherein the levels of a substance that is affected by the therapeutic drug are continuously monitored.

50. The method of claim 49, wherein the substance is glucose and the therapeutic drug is insulin.

51. The method of claim 35, further including storing the levels of the therapeutic drug, the metabolite of the therapeutic drug or the substance that is affected by the therapeutic drug.

52. The method of claim 35, wherein the therapeutic drug is a water-soluble drug.

53. The method of claim 35, wherein the therapeutic drug is a water-insoluble drug.
